Europäisches Patentamt

**(19)** European Patent Office

Office européen des brevets

**(11)** **EP 1 173 137 B1**

**(12)** **EUROPÄISCHE PATENTSCHRIFT**

**(45)** Veröffentlichungstag und Bekanntmachung des Hinweises auf die Patenterteilung:
**01.12.2004  Patentblatt 2004/49**

**(51)** Int Cl.⁷: **A61K 6/087**, A61K 6/083

**(21)** Anmeldenummer: **00929416.6**

**(86)** Internationale Anmeldenummer:
**PCT/EP2000/003676**

**(22)** Anmeldetag: **25.04.2000**

**(87)** Internationale Veröffentlichungsnummer:
**WO 2000/066069 (09.11.2000 Gazette 2000/45)**

**(54)** **DENTALMATERIALIEN**

DENTAL MATERIALS

MATERIAUX DENTAIRES

**(84)** Benannte Vertragsstaaten:
**AT BE CH CY DE DK ES FI FR GB GR IE IT LI LU MC NL PT SE**

**(30)** Priorität: **29.04.1999  DE 19919581**

**(43)** Veröffentlichungstag der Anmeldung:
**23.01.2002  Patentblatt 2002/04**

**(73)** Patentinhaber: **3M ESPE AG**
**82229 Seefeld (DE)**

**(72)** Erfinder:
  • **ECKHARDT, Gunther**
    **82346 Frieding (DE)**
  • **LUCHTERHANDT, Thomas**
    **86926 Greifenberg (DE)**
  • **KLETTKE, Thomas**
    **82229 Hechendorf (DE)**

**(56)** Entgegenhaltungen:
  **EP-A- 0 985 684**      **WO-A-98/17694**
  **DE-A- 3 201 780**      **DE-A- 3 939 164**
  **DE-A- 19 736 471**     **GB-A- 1 113 722**

## Beschreibung

Seit einiger Zeit wird in der Dentalmedizin der Ersatz des klassischen Füllungsmaterials Amalgam durch Composite ("Kunststoff") angestrebt. Im Gegensatz zu Amalgam ist es bei der Versorgung mit Compositen notwendig, einen Haftvermittler zu verwenden. Dieses sogenannte Bonding wird vor dem Einbringen des Composites in die Kavität auf die präparierte Zahnhartsubstanz aufgebracht.

Eine wesentliche Forderung der Anwender von Dentalmaterialien besteht darin, dass die Verarbeitung möglichst einfach sein soll, keine Verarbeitungsfehler zulässt und immer zum gleichen, gewünschten Resultat führt.

Dieser Forderung kann zumindest teilweise entsprochen werden, wenn einkomponentige Dentalmaterialien hergestellt und verarbeitet werden.

Die Anwendung von einkomponentigen Dentalmaterialien setzt voraus, dass die Auslösung der polymerbildenden Reaktion entweder durch Zuführung von Energie und bzw. oder durch Reaktion mit Bestandteilen "aktiver" Oberflächen oder der umgebenden Atmosphäre erfolgen kann.

Ein typischer Fall für die Polymerisationsauslösung durch Energiezufuhr ist die photoinduzierte Härtung von Dentalmaterialien, bei der durch Bestrahlung mit Licht der Zerfall von Photoinitiatoren in polymerisationsauslösende Spezies bewirkt wird.

Die Aushärtegeschwindigkeit solcher lichthärtender Dentalmaterialien ist im allgemeinen sehr hoch, jedoch findet die Aushärtung nur in den Bereichen statt, die vom Licht erreicht werden.

Wegen der geringen Lebensdauer der polymerisationsauslösenden Spezies, wie der freien Radikale, ist es meist nur möglich, die Belichtung und damit die Aushärtung am Ort der Endanwendung des ausgehärteten Dentalmaterials zu realisieren.

Dieser Umstand schränkt die Anwendung der Lichthärtung von Dentalmaterialien ein. Gewünscht werden deshalb Dentalmaterialien, die als einkomponentige Zubereitungen, eine ausreichende Lagerbeständigkeit besitzen und die in einfacher Weise "aktivierbar" sind.

Unter "Aktivierbarkeit" wird hierbei verstanden, dass in den aus dem Lagerbehälter ausgebrachten Dentalmaterialien durch Energieeintrag und bzw. oder durch Reaktion mit Bestandteilen "aktiver" Oberflächen oder der umgebenden Atmosphäre polymerisationsauslösende Spezies erzeugt werden mit der Maßgabe, dass die Dentalmaterialien nach der "Aktivierung" mindestens noch 10 Sekunden ausreichend fließfähig sind, um eine bestimmungsgemäße Verwendung zu gewährleisten.

Die GB 1 113 722 A betrifft die Polymerisation von Acrylaten unter Verwendung von Triarylborankomplexen. Es wird ausgeführt, dass es vorteilhaft sein kann, wenn bestimmte Zusammensetzungen eine bestimmte Menge von Oxirangruppen enthalten, um die Klebeeigenschaften zu verbessern.

DE 39 39 164 A beschreibt neue Startersysteme für die Polymerisationsauslösung ethylenisch ungesättigter Verbindungen und deren Verwendung u. a. in der Zahnmedizin. Das beschriebene Startersystem basiert auf Sauerstoff-reaktiven Organo-Borverbindungen. Als polymerisierbare Monomere sind (Meth)acrylatverbindungen in Kombination mit (Meth)acrylsäure genannt.

Gegenstand von WO 98 17694 A ist eine zweikomponentige, härtbare Zusammensetzung, enthaltend einen Organo-Boran-Amin-Komplex und ein Aziridin-funktionelles Material.

Es besteht demnach die Aufgabe, lagerstabile einkomponentige Dentalmaterialien vorzuschlagen, die nach einer "Aktivierung" eine ausreichend lange Verarbeitungszeit aufweisen.

Die Aufgabe der Erfindung wird gelöst durch Dentalmaterialien, enthaltend Monomere und bzw. oder Prepolymere, die zu einer polymerbildenden Reaktion befähigt sind, mindestens ein erstes und ein zweites Initiierungssystem, sowie ggf. Füllstoffe, Farbstoffe, Fließmodifikatoren, Stabilisatoren, Ionen-abgebende Substanzen, die Röntgenopazität erhöhende Verbindungen oder weitere Modifikatoren, wie die in den Ansprüchen beschrieben sind.

Die dabei entstehende Schmierschicht ist vorzugsweise kleiner als 0,2 mg/cm$^2$.

Erfindungsgemäß wird eine sauerstoffempfindliche Verbindung eingesetzt, die bei Inkontaktbringen mit Sauerstoff eine angeregte oder reaktive Spezies, vorzugsweise Radikale, bilden kann, die ihrerseits über eine weitere Reaktionsfolge aus einem salzartigen Initiator Säure freisetzen kann, welche eine Polymerisationsreaktion, insbesondere eine kationische Polymerisationsreaktion auslösen kann.

Bei diesem salzartigem Initiator handelt es sich um Iodonium-Verbindungen, die, wenn beispielsweise radikalisch aktiviert, in Säuren zerfallen können.

Somit stehen für die Steuerung des Ablaufs der Polymerisationsreaktion zwei miteinander reagierende Initiatorsysteme zur Verfügung.

Nach einer bevorzugten Ausführungsform der Erfindung werden die Dentalmaterialien aus dem dichten, sauerstoffundurchlässigen Lagerbehälter ausgebracht und mit der Umgebungsluft innerhalb eines Zeitraumes von 1 bis 120 Sekunden in Kontakt gebracht. Dabei kann es zweckmäßig sein, durch Ausstreichen und leichtes Mischen neue Oberflächen zu schaffen, was die "Aktivierung" beschleunigt.

Nach einer weiteren Ausführungsform der Erfindung werden die Dentalmaterialien auf "aktive" Oberflächen aufgebracht, die Sauerstoff abgeben, wobei der Sauerstoff beispielsweise durch Reaktion einer auf der Oberfläche befindlichen Verbindung mit einem Bestandteil der flüssigen Dentalmaterialien erzeugt werden kann.

Die erfindungsgemäßen Dentalmaterialien härten unter Polymerbildung aus, wobei bevorzugt solche Monomere bzw. Prepolymere eingesetzt werden, die nach einem Kationenkettenmechanismus, durch einen Thiol-en-Mechanismus oder nach einem Radikalkettenmechanismus polymerisieren.

Kombinationen mehrerer Mechanismen sind ebenfalls möglich.

So können die Dentalmaterialien Monomere und Prepolymere enthalten, die durch ringöffnende Polymerisation aushärten, wobei die Verwendung von Verbindungen, die mindestens zwei Epoxidgruppen enthalten, bevorzugt ist. Cycloaliphatische Epoxidharze, die nach einem kationischen Ringöffnungsmechanismus aushärten, stellen eine mit Vorteilen anwendbare Monomerklasse dar.

Typische Vertreter dieser cycloaliphatischen Epoxidharze sind beispielsweise in DE 196 48 283 A1 beschrieben.

Die Dentalzubereitungen, die nach einem Thiol-en-Mechanismus aushärten, enthalten Multi-Thiol-Verbindungen, wie das Tetramercaptopropionat des Pentaerythrits, und Multiallylverbindungen, wie Triallylisocyanurat.

Solche Dentalmaterialien auf der Grundlage von Thiol-en-Systemen sind in DE-A-3837569, auf die hier Bezug genommen wird, ausführlich beschrieben.

Typische Monomere bzw. Prepolymere, die nach einem Radikalkettenmechanismus aushärten und in Dentalmaterialien eingesetzt werden, sind Acrylate oder Methacrylate. Geeignet sind allgemein ein- und mehrfunktionelle (Meth)acrylatmonomere. Typische Vertreter dieser Verbindungsklasse sind Alkyl(meth)acrylate, einschließlich der Cycloalkyl(meth)acrylate, Aralkyl(meth)-acrylate und 2-Hydroxyalkyl(meth)acrylate, wie Hydroxypropylmethacrylat, Hydroxyethylmethacrylat, Isobomylacrylat, Isobomylmethacrylat, Butylglycolmethacrylat, Acetylglykolmethacrylat, Triethylenglycoldimethacrylat, Polyethylenglycoldimethacrylat, 2-Phenylethylmethacrylat, 2-Ethylhexylmethacrylat, Cyclohexylmethacrylat, Laurylmethacrylat und Hexandioldi(meth)acrylat, wie es in der DE-A-4328960 beschrieben wird.

Verwendet werden können auch langkettige Monomere auf der Basis von Bisphenol A und Glycidylmethacrylat oder deren durch Addition von Isocyanaten entstandene Derivate, wie es in der US-A-3066112 beschrieben wird. Geeignet sind auch Verbindungen des Typs Bisphenyl-A-diethyloxy(meth)acrylat und Bisphenol-A-dipropyloxy(meth)acrylat.

Weiterhin Verwendung finden können die oligoethoxylierten und oligopropoxylierten Bisphenol-A-diacryl- und -dimethacrylsäureester. Gut geeignet sind außerdem die in der DE-C-2816823 genannten Diacryl- und Dimethacrylsäureester des Bis(hydroxymethyl)-tricyclo$[5.2.1.0^{2,6}]$-decans und die Diacryl- und Dimethacrylsäureester der mit 1 bis 3 Ethylenoxid- und/oder Propylenoxideinheiten verlängerten Verbindungen des Bis(hydroxymethyl)-tricyclo$[5.2.1.0^{2,6}]$-decans. Diese Aufzählung ist beispielhaft und in keiner Weise abschließend zu verstehen.

Es können auch Gemische der genannten Monomeren verwendet werden.

Die erfindungsgemäßen Dentalmaterialien enthalten mindestens ein erstes Initiierungssystem, das bei Kontakt mit Sauerstoff die polymerbildende Reaktion auslösende Spezies freisetzt.

Bevorzugt enthalten die Dentalmaterialien Verbindungen, die durch Sauerstoff schnell oxidiert werden, wobei durch diese Reaktion oder durch Folgereaktionen freie Radikale gebildet werden, die entweder direkt die Polymerbildung einleiten oder nach Reaktion mit weiteren Bestandteilen der flüssigen Dentalzubereitungen die die polymerbildenden Reaktionen auslösenden Spezies bilden.

Geeignete Verbindungsklassen, die einer schnellen Oxidation durch Sauerstoff unterliegen, sind beispielsweise substituierte Hydrazone und Borane.

So wird die Autoxidation von Hydrazonen bereits in den Ber. 47 (1994) 3277 bis 3291 beschrieben.

Hydrazone enthaltende, durch Zutritt von Luftsauerstoff radikalisch polymerisierbare Mehrstoffgemische werden in der DE-A-4000776, Spalte 2 (Zeile 67) bis Spalte 9 (Zeile 41) beschrieben, worauf zur Offenbarung ausdrücklich Bezug genommen wird.

Vorzugsweise können auch Hydrazon-Verbindungen eingesetzt werden, die in EP-A-0510035 und in EP-A-0594671 beschrieben sind. Diese Hydrazone sind zur Ausbildung von Hydroperoxiden befähigt und werden üblicherweise als Polymerisationsstarter für radikalisch polymerisierbare Monomere eingesetzt.

In der vorliegenden Erfindung kommen Hydrazone der beschriebenen Art zusammen mit Iodonium-Verbindungen, insbesondere Bisaryliodoniumsalzen starker Säuren, zur Aushärtung von Epoxidgruppen-haltigen Monomere enthaltenden Zubereitungen zum Einsatz.

Die DE-A-3041904 beschreibt Borverbindungen enthaltende, nach Sauerstoff-Kontakt härtende Kunststoffmassen, die insbesondere als Reaktionsklebstoffe eingesetzt werden.

in der DE-A-3201780 werden neue polymere Organo-Borverbindungen vorgeschlagen, die den radikalisch polymerisierbaren Mehrstoffgemischen bei hoher initiierender Wirkung eine verbesserte Stabilität verleihen.

Die EP-A-0835646 beschreibt Klebstoffzusammensetzungen, die organische Borverbindungen enthalten und unter anderem in Dentalmaterialien, wie Bondings eingesetzt werden können.

Den vorstehend genannten Lösungsvorschlägen ist gemeinsam, dass sie sich ausschließlich auf radikalisch härtende Zubereitungen beziehen und die wesentliche Forderung der Anwender von Dentalmaterialien nach möglichst einfacher Verarbeitung und Konstanz der Verarbeitungsergebnisse sehr eingeschränkt erfüllt wird.

Die erfindungsgemäßen Zubereitungen und Verarbeitungsverfahren erweitern die möglichen Polymerbildungsreaktionen um die kationische Ringöffnungspolymerisation und um Thiol-en-Systeme, wobei eine ausreichende Lagerbeständigkeit und eine einfache Verarbeitbarkeit erreicht wird.

In radikalisch polymerisierenden Zubereitungen werden Eigenschaften erreicht, die über die im Stand der Technik beschriebenen weit hinausgehen.

Bei Verwendung von Monomeren bzw. Prepolymeren, die durch eine kationische Ringöffnungspolymerisation aushärten, ist zusätzlich zu den mit Sauerstoff reagierenden Verbindungen, wie den Hydrazonen oder Boranen, die Anwesenheit von Verbindungen erforderlich, die unter Einwirkung von Radikalen unter Bildung von Säuren zerfallen.

Als besonders geeignet, haben sich für diese Ausführungsform der Erfindung Säurebildner erwiesen, wie sie in der DE-A-19736471 ausführlich beschrieben werden.

Die erfindungsgemäßen Dentalmaterialien enthalten, bezogen auf die Gesamtzubereitung, üblicherweise 0,1 bis 10 Gew.-%, vorzugsweise 0,2 bis 5 Gew.-% und besonders bevorzug 0,5 bis 3 Gew.-% Initiator und, bezogen auf die Gesamtzubereitung, üblicherweise 90 bis 99,9 Gew.-%, vorzugsweise 95 bis 99,8 Gew.-% und besonders bevorzugt 97 bis 99,5 Gew.-% Monomere oder Präpolymere oder deren Mischungen.

Bei Verwendung dieser Kombination von Verbindungen ist auch die Aushärtung von Monomergemischen, die sowohl radikalisch als auch kationisch polymerisierbare Substanzen enthalten, möglich.

Die erfindungsgemäßen Dentalmaterialien können ggf. Füllstoffe, Farbstoffe, Fließmodifikatoren, Stabilisatoren, Ionen-abgebende Substanzen, die Röntgenopazität erhöhende Verbindungen oder weitere Modifikatoren enthalten.

Als Füllstoffe sind bespielsweise Stoffe geeignet, wie sie in der DE-A-19648283 (Seite 10, Zeile 48-59) beschrieben sind.

Wenn die erfindungsgemäßen Dentalmaterialien Füllstoffe enthalten, umfassen sie:

a) vorzugsweise 0,1 bis 10 Gew.-%, in bevorzugterer Weise 0,2 bis 5 Gew.-% und in besonders bevorzugter Weise 0,5 bis 3 Gew.-% Initiator,

b) vorzugsweise 3 bis 84,9 Gew.-%, in bevorzugterer Weise 5 bis 75 Gew.-% und in besonders bevorzugter Weise 20 bis 75 Gew.-% Füllstoffe,

c) vorzugsweise 15 bis 96,9 Gew.-%, in bevorzugterer Weise 20 bis 94,8 Gew.-% und in besonders bevorzugter Weise 22 bis 79,5 Gew.-% Monomere oder Prepolymere oder deren Mischungen.

Wenn die Zusammensetzungen darüber hinaus einen oder mehrere der oben erwähnten weiteren Zusatzstoffe, wie Farbstoffe, Fließmodifikatoren etc., enthalten, sind diese in auf dem Dentalgebiet üblichen Mengen vorhanden.

Ein besonderer Vorteil der erfindungsgemäßen Zubereitungen und Verarbeitungsverfahren besteht darin, dass die Bereiche der "Aktivierungszeit" und der "Verarbeitungszeit" durch die Zusammensetzung der aktivierbaren Dentalmaterialien vorgegeben sind und durch den Verarbeiter die notwendige "Verarbeitungszeit" durch die Intensität des Inkontaktbringens mit Sauerstoff bzw. Luft in einem vorgegebenen Rahmen beeinflusst werden kann.

Die erfindungsgemäßen Dentalmaterialien werden für ihren bestimmungsgemäßen Gebrauch in geeignete Behältnisse abgepackt, die vorzugsweise Sauerstoff- und lichtundurchlässig sind. Als Behältnisse kommen Kartuschen, Mischkapseln, Compules, oder Tuben in Frage.

Beispiele

Ausführungsbeispiel 1 :

In einem Dreifingerkneter werden unter Stickstoff im Dunkelraum die nachfolgend beschriebenen Substanzen zu einer homogenen Paste geknetet.

Die Paste wird evakuiert, das Vakuum durch Stickstoff aufgefüllt und die Paste in lichtundurchlässige sowie sauerstoffundurchlässige Dosierbehälter gebracht.

| Zusammensetzung der Paste: | Gramm auf 100g Paste |
|---|---|
| Bis(4-Dodecylphenyl)iodoniumtetrakis (pentafluorophenyl)borat | 0,62 |

(fortgesetzt)

| Zusammensetzung der Paste: | Gramm auf 100g Paste |
|---|---|
| 3, 4-Epoxycyclohexylmethyl-3', 4'-epoxycyclohexylcarboxylat | 14, 19 |
| 1, 3, 5, 7-Tetrakis-(2, 1-ethandiyl-3, 4-epoxycyclohexyl)- 1, 3, 5, 7-tetramethylcyclotetrasiloxan | 7,81 |
| Quarz, mittlere Korngröße 2 Mikrometer, silanisiert mit Epoxysilan | 66,51 |
| Tributylboran* | 0,47 |
| 2-Butoxyethyl-4-dimethylaminobenzoat | 0,08 |
| Dilithiumcarboxylat einer Polyethylenglycol-600-disäure | 0,11 |
| Bisphenol-A-monoglycidethermonomethacrylat (UVACURE 1561) | 10,21 |

* Polymeres Boralkyl B2, hergestellt gemäß den Angaben in Tabelle B der DE-A-3201780.

Die Paste wurde zur Zementierung von Kronen verwendet.

Hierzu wurden aus dem Dosierbehälter 0,3 g der Paste auf einen Silikonpapier-Block ausgebracht, 20 Sekunden mit dem Spatel leicht durchgeknetet, in die Krone eingefüllt und die gefüllte Krone auf den präparierten Stumpf aufgesetzt.

Die überstehende Paste konnte nach 2 Minuten leicht entfernt werden.

Die zementierte Krone war 10 Minuten nach dem Aufsetzen auf den Stumpf funktionsfähig.

In lichtundurchlässigen und sauerstoffundurchlässigen Dosierbehältem war die Paste über einen Zeitraum von 18 Monaten lagerstabil.

Eine andere Anwendung der erfindungsgemäßen Zubereitungen liegt im Bereich der provisorischen Kronen und Brücken. Bei ihrer Herstellung geht der Zahnarzt üblicherweise so vor, dass er vor der Präparation von der momentanen Zahnsituation, beispielsweise mit einem Silikon-Präzisionsabformmaterial, einen Abdruck nimmt.

Nach der Präparation des Zahnes bzw. der Zähne benötigen diese einen provisorischen Schutz und werden mit einer provisorischen Krone bzw. Brücke versehen. Dazu wird der Situationsabdruck mit einem entsprechenden Material, dem angemischten provisorischen Kronen- und Brückenmaterial, ausgefüllt. Der gefüllte Abdruck wird sodann auf die präparierten Zahnstümpfe gegeben und im Mund bis zur elastischen Phase ausgehärtet. Während der elastischen Phase wird das Material entnommen und bis zur vollständigen Aushärtung bei Raumtemperatur belassen. Nach der Aushärtung befindet sich üblicherweise auf dem Material eine sogenannte "Schmierschicht". Diese wird meist mit der Inhibierung der radikalischen Polymerisation an der Oberfläche durch Sauerstoff in Verbindung gebracht.

Bevor der Zahnarzt das Provisorium weiter bearbeiten kann, muss die Schmierschicht entfernt werden, was einen erheblichen Arbeitsaufwand bedeutet. Bei Anwendung der erfindungsgemäßen Zubereitungen zeigte sich, dass praktisch keine Schmierschicht auftritt und damit dieser erhebliche Nachteil beseitigt worden ist. Die nachfolgend aufgeführten Zubereitungen sind Ausführungsbeispiele und in keiner Weise limitierend zu verstehen.

Ausführungsbeispiel 2 (Referenz)

**Zweikomponentiges Material für provisorische Kronen und Brücken, welches im Verhältnis 10 / 1 (Basis / Katalysator) angemischt wird und schmierschichtfrei aushärtet.**

Die Basispaste sowie die Katalysatorpaste werden aus den in Tabelle 1 genannten Bestandteilen unter Stickstoff durch Kneten angemischt.

| Basispaste: | Gramm auf 100 g Paste: |
|---|---|
| - Pyrogene Kieselsäure (Korngröße < 0,05 Mikrometer) | 5.00 |
| - Glaspulver, silanisiert (mittlere Korngröße 10 Mikrometer) | 32.50 |
| - 2,2-Bis(4-(oligo(ethoxy))-phenyl)-propandimethacrylat | 61.48 |
| - N,N-Dimethyl-p-toluidin | 0.50 |
| - Hydrochinonmonomethylether | 0.02 |
| - Tributylboran * | 0.50 |
| Katalysatorpaste: | Gramm auf 100 g Paste |
| - Glaspulver, silanisiert | 32.00 |

* Polymeres Boralkyl B2, hergestellt gemäß den Angaben in Tabelle B der DE-A-3201780.

(fortgesetzt)

| Katalysatorpaste: | Gramm auf 100 g Paste |
|---|---|
| (mittlere Korngröße 10 Mikrometer) -2,2-Bis(4-(oligo(ethoxy))-phenyl)-propandiacetat | 65.50 |
| - Benzoylperoxid (Peroxid Chemie) | 2.50 |

Bei Verwendung der provisorischen Kronen- und Brücken-Zubereitung nach Ausführungsbeispiel 2 ergibt sich eine "Gesamtschmierschicht", für die ein Zahlenwert von < 0,2 mg / cm$^2$, entsprechend der Nachweisgrenze der unten beschriebenen Methode, ermittelt wurde.

Vergleichsbeispiel 1:

Ein handelsübliches, radikalisch härtendes Provisorienmaterial zeigt bei Verwendung eines Silikon-Abdruckmaterials, sowohl auf dem Provisorium (ca. 1.6 mg / cm$^2$) als auch auf dem Abdruckmaterial (ca. 1.2 mg / cm$^2$), eine Schmierschicht (Bestimmung siehe unten). Es ergibt sich für die "Gesamtschmierschicht" ein Zahlenwert von ca. 2.8 mg / cm$^2$.

Methode zur Bestimmung der Schmierschicht

Die Schmierschicht wird folgendermaßen bestimmt:

Ein Delrinring (DuPont) (d = 20 mm; h = 3,5 mm ) wird auf ein Plättchen aus Abformmaterial (AP) gelegt, blasenfrei mit Probenmaterial (PM) befüllt, mit einem Objektträger abgedeckt, und 1h bei RT ausgehärtet. Nach Entfernung des Objektträgers erfolgt Herausdrücken des Prüfkörpers und Wägung von PM und AP mit Schmierschicht (ergibt m1 und m2)

Danach wird die Schmierschicht von AP und PM mit ethanolfeuchtem Papier entfernt. Die Wägung ergibt die Massen von AP und PP ohne Schmierschicht (m3 und m4).

Die Gesamtschmierschicht S ergibt sich aus: $S = \dfrac{m(a)-m(b)[mg]}{F[cm^2]}$

m(a): m1 + m2 (Gesamtmasse (AP+ PP) mit Schmierschicht)

m(b): m3 + m4 (Gesamtmasse (AP+ PP) ohne Schmierschicht)

Fläche $F = \dfrac{d^2[cm]*\pi}{4} = 3{,}142\ cm^2$

Ausführungsbeispiel 3

**Zweikomponentiges Material für provisorische Kronen und Brücken, welches im Verhältnis 10 / 1 (Basis / Katalysator) angemischt wird und schmierschichtfrei aushärtet.**

Die Herstellung der Zubereitung erfolgte wie unter Ausführungsbeispiel 2 beschrieben.

| Basispaste: | Gramm auf 100 g Paste |
|---|---|
| - Pyrogene Kieselsäure, (Korngröße < 0,05 Mikrometer) | 5.00 |
| - Glaspulver, silanisiert (mittlere Korngröße 10 Mikrometer) | 32.50 |
| - 2,2-Bis(4-(oligo(ethoxy))-phenyl)-propandimethacrylat | 49.00 |
| - Bisphenol-A-monoglycidethermonomethacrylat (UVACURE 1561, Fa. UCB) | 12.00 |
| - Tributylboran* | 1.00 |
| - Diphenyliodoniumhexafluorophosphat (Fa. Avocado) | 0.50 |
| Katalysatorpaste: | Gramm auf 100g Paste: |
| - Glaspulver, silanisiert (mittlere Korngröße 10 Mikrometer) | 32.00 |
| - 2,2-Bis(4-(oligo(ethoxy))-phenyl)-propan-diacetat | 65.00 |
| - Benzoylperoxid (Peroxid Chemie) | 2.50 |

* Polymeres Boralkyl B2, hergestellt gemäß den Angaben in Tabelle B der DE-A-3201780.

Bei Verwendung der Zubereitung gemäß Ausführungsbeispiel 3 ergibt sich eine "Gesamtschmierschicht", für die ein Zahlenwert von < 0,2 mg / cm$^2$ ermittelt wurde.

**Patentansprüche**

1. Dentalmaterialien, enthaltend epoxidgruppenhaltige Monomere und bzw. oder Prepolymere, die nach einem Kationenkettenmechanismus aushärten mindestens ein erstes und ein zweites Initiierungssystem, sowie ggf. Füllstoffe, Farbstoffe, Fließmodifikatoren, Stabilisatoren, Ionenabgebende Substanzen, die Röntgenopazität erhöhende Verbindungen oder weitere Modifikatoren, wobei
das erste Initiierungssystem Borane und-/oder Hydrazone enthält und so beschaffen ist, dass es beim Inkontaktbringen mit Sauerstoff die polymerbildende Reaktion auslösende Spezies freisetzt, das zweite Initiierungssystem radikalisch spaltbare Iodoniumverbindungen enthält und die Menge der Initiierungssysteme so bemessen ist, dass die Dentalmaterialien nach dem Inkontaktbringen mit Sauerstoff noch mindestens 10 Sekunden ausreichend fließfähig sind-und danach zu einem Feststoff aushärten.

2. Dentalmaterialien gemäß Anspruch 1, wobei die Dentalmaterialien nach dem Inkontaktbringen mit Luft aushärten.

3. Dentalmaterialien gemäß einem der Ansprüche 1 oder 2, wobei die Dentalmaterialien doppelbindungshaltige Monomere enthalten.

4. Dentalmaterialien gemäß einem der Ansprüche 1 oder 2, wobei die Dentalmaterialien Monomere und bzw. oder Prepolymere enthalten und durch eine kationische Ringöffnungspolymerisation aushärten.

5. Dentalmaterialien gemäß einem der Ansprüche 1 oder 2, wobei die Dentalmaterialien sowohl epoxidgruppenhaltige als auch doppelbindungshaltige Monomere und bzw. oder Prepolymere enthalten.

6. Dentalmaterialien gemäß einem der Ansprüche 1 oder 2, wobei die 5 Dentalmaterialien (meth-)acrylgruppenhaltige Monomere und Prepolymere enthalten und durch einen Radikalkettenmechanismus aushärten.

7. Dentalmaterialien gemäß einem der Ansprüche 1 oder 2, wobei die Dentalmaterialien Allylgruppen-haltige Monomere oder Norbomengruppenhaltige Monomere und Multi-Thiol-Verbindungen enthalten und nach einem Thiolen-Mechanismus aushärten.

8. Dentalmaterialien nach einem der Ansprüche 1 bis 7, wobei die Dentalmaterialien Diaryliodoniumverbindungen enthalten.

9. Verfahren zur Aushärtung von Dentalmaterialien gemäß den Ansprüchen 1 bis 8, wobei die Dentalmaterialien vor ihrer Anwendung in licht- sowie sauerstoffdicht schließenden Behältern aufbewahrt werden, die Dentalmasse aus diesem Behälter ausgebracht und innerhalb eines Zeitraumes von 1 bis 120 Sekunden mit der Umgebungsluft in Kontakt gebracht wird.

10. Verfahren zur Aushärtung von Dentalmaterialien, gemäß den Ansprüchen 1 bis 8, wobei der Sauerstoff durch eine chemische Reaktion mit einer "aktiven Oberfläche in den Dentalmaterialien erzeugt wird.

11. Verfahren zur Aushärtung von Dentalmaterialien, gemäß den Ansprüchen 1 bis 8 und Anspruch 10, wobei eine "aktive" Oberfläche vor dem Ausbringen des Dentalmaterials durch Dotieren einer Oberfläche mit einem sauerstoffreichen Material erzeugt wird.

12. Verfahren zur Aushärtung von Dentalmaterialien gemäß den Ansprüchen 1 bis 8, wobei die Dentalmaterialien pastenförmig sind und nach dem Ausbringen auf eine Unterlage mit einem Handgerät innerhalb eines Zeitraumes von 1 bis 60 Sekunden unter Bildung neuer Oberflächen mit Luft in Kontakt gebracht werden.

13. Verfahren zur Aushärtung von Dentalmaterialien gemäß den Ansprüchen 1 bis 8, wobei die Dentalmaterialien leichtfließend und ggf. lösemittelhaltig sind und nach dem Ausbringen mit einem Luftstrom behandelt werden.

14. Verwendung von Dentalmaterialien gemäß den Ansprüchen 1 bis 8 zur Herstellung eines materials, geeignet zur Befestigung von Kronen, Brücken und Inlays, Füllung von Kavitäten, Behandlung von mechanisch präparierten Kavitäten, Behandlung von Kavitäten, die durch einen Ätzschritt vorbehandelt sind, als bindende Zwischenschicht zwischen einer vorbehandelten Kavitätenwand und einem Füllmaterial.

15. Dentalmaterialien, enthaltend epoxidgruppenhaltige Monomere und bzw. oder Prepolymere, die nach einem Kationenkettenmechanismus aushärten, mindestens ein erstes und ein zweites Initiierungssystem, sowie ggf. Füllstoffe, Farbstoffe, Fließmodifikatoren, Stabilisatoren, Ionen-abgebende Substanzen, die Röntgenopazität erhöhende Verbindungen oder weitere Modifikatoren, wobei das erste Initiierungssystem Borane und/oder Hydrozone enthält und so beschaffen ist, dass es beim Inkontaktbringen mit Sauerstoff die polymerbildende Reaktion auslösende Spezies freisetzt, das zweite Initiierungssystem radikalisch spaltbare Iodoniumverbindungen enthält und die Menge der Initiierungssysteme so bemessen ist, dass die Dentalmaterialien nach dem Inkontaktbringen mit Sauerstoff zu einem Feststoff aushärten, wobei die unpolymerisierte Monomerschicht auf der Oberfläche (Schmierschicht) kleiner als 0,2 mg / cm$^2$ ist.

16. Dentalmaterialien nach Anspruch 15, wobei die Menge des Initiierungssystems so bemessen ist, dass die Dentalmaterialien nach dem Inkontaktbringen mit Sauerstoff noch mindestens 10 Sekunden ausreichend fließfähig sind und danach zu einem Feststoff aushärten.

17. Behältnis, enthaltend ein Dentalmaterial nach einem der Ansprüche 1 bis 8 oder 15 bis 16.

18. Verwendung der Dentalmaterialien gemäß den Ansprüche 15 oder 16 zur Herstellung von provisorischen Kronen und Brücken.

19. Verwendung eines Systems, umfassend mindestens ein erstes -und ein zweites Inititierungsystem, umfassend mindestens eine sauerstoffempfindliche Substanz gewählt aus Boranen und/oder Hydrazonen und mindestens eine Verbindung, die unter Einwirkung von Radikalen unter Bildung von Säuren zerfällt gewählt aus Iodonium-Verbindungen zur Aushärtung von polymerbildenden Zubereitungen.

20. Verwendung eines Systems nach Anspruch 19, wobei die Verbindung, die unter Einwirkung von Radikalen unter Bildung von Säuren zerfällt, gewählt ist aus Diaryliodonium-Verbindungen.

21. Verwendung eines Systems nach einem der Ansprüche 19 bis 20, wobei die polymerbildende Zubereitung epoxidhaltige Substanzen umfasst.

22. Verwendung eines Systems nach einem der Ansprüche 19-21 zur Einstellung der Verarbeitungszeit eines Dentalmaterials.

**Claims**

1. Dental materials, comprising
monomers and/or prepolymers which comprise epoxide groups and which cure according to a cation chain mechanism,
at least one first and one second initiating system,
and, if appropriate, fillers, colorants, flow modifiers, stabilizers, ion-releasing substances, compounds which increase the opaqueness to X-rays, or additional modifiers, in which
the first initiating system comprises boranes and/or hydrazones and is provided in such a way that it releases the entity which initiates the polymer-forming reaction when brought into contact with oxygen, the second initiating system comprises radically cleavable iodonium compounds and the amount of the initiating systems is calculated in such a way that the dental materials, after being brought into contact with oxygen, are sufficiently flowable for at least 10 seconds still and afterwards cure to give a solid.

2. Dental materials according to Claim 1, in which the dental materials cure after being brought into contact with air.

3. Dental materials according to either of Claims 1 and 2, in which the dental materials comprise monomers possessing a double bond.

4. Dental materials according to either of Claims 1 and 2, in which the dental materials comprise monomers and/or prepolymers and cure by cationic ring-opening polymerization.

5. Dental materials according to either of Claims 1 and 2, in which the dental materials comprise monomers and/or prepolymers which comprise both epoxide groups and a double bond.

6. Dental materials according to either of Claims 1 and 2, in which the dental materials comprise monomers and prepolymers which comprise (meth)acryl groups and cure by a radical chain mechanism.

7. Dental materials according to either of Claims 1 and 2, in which the dental materials comprise monomers which comprise allyl groups or comprise monomers which comprise norbornene groups and comprise polythiol compounds and cure according to a thiol-ene mechanism.

8. Dental materials according to one of Claims 1 to 7, in which the dental materials comprise diaryliodonium compounds.

9. Process for curing dental materials according to Claims 1 to 8, in which the dental materials, before they are used, are kept in lightfast and oxygenfast closed containers, the dental composition is applied from this container and is brought into contact with the surrounding air inside a time period of 1 to 120 seconds.

10. Process for curing dental materials according to Claims 1 to 8, in which the oxygen is produced by a chemical reaction with an "active" surface in the dental materials.

11. Process for curing dental materials according to Claims 1 to 8 and Claim 10, in which an "active" surface is produced before the dental material is applied by doping a surface with an oxygen-rich material.

12. Process for curing dental materials according to Claims 1 to 8, in which the dental materials are paste-like and, after the application to a substrate with a manual device, are brought into contact with air inside a time period of 1 to 60 seconds with formation of fresh surfaces.

13. Process for curing dental materials according to Claims 1 to 8, in which the dental materials are easy-flowing and, if appropriate, solvent-comprising and, after the application, are treated with a stream of air.

14. Use of dental materials according to Claims 1 to 8 for the preparation of a material, suitable for the attachment of crowns, bridges and inlays, filling of cavities, treatment of mechanically prepared cavities or treatment of cavities, which are pretreated through an etching stage, as binding intermediate layer between a pretreated cavity wall and a filling material.

15. Dental materials, comprising monomers and/or prepolymers which comprise epoxide groups and which cure according to a cation chain mechanism, at least one first and one second initiating system, and, if appropriate, fillers, colorants, flow modifiers, stabilizers, ion-releasing substances, compounds which increase the opaqueness to X-rays, or additional modifiers, in which the first initiating system comprises boranes and/or hydrazones and is provided in such a way that it releases the entity which initiates the polymer-forming reaction when brought into contact with oxygen, the second initiating system comprises radically cleavable iodonium compounds and the amount of the initiating systems is calculated in such a way that the dental materials, after being brought into contact with oxygen, cure to give a solid, the unpolymerized monomer layer on the surface (lubricating film) being less than 0.2 mg/cm$^2$.

16. Dental materials according to Claim 15, in which the amount of the initiating system is so calculated that the dental materials, after being brought into contact with oxygen, are sufficiently flowable for at least 10 seconds still and afterwards cure to give a solid.

17. Container, comprising a dental material according to one of Claims 1 to 8 or 15 to 16.

18. Use of the dental material according to Claim 15 or Claim 16 for the preparation of temporary crowns and bridges.

19. Use of a system, comprising at least one first and one second initiating system, comprising at least one oxygen-sensitive substance chosen from boranes and/or hydrazones and at least one compound chosen from iodonium compounds which decomposes under the action of radicals with the formation of acids, for the curing of polymer-forming preparations.

**20.** Use of a system according to Claim 19, in which the compound which decomposes under the action of radicals with the formation of acids is chosen from diaryliodonium compounds.

**21.** Use of a system according to either of Claims 19 and 20, in which the polymer-forming preparation comprises epoxide-comprising substances.

**22.** Use of a system according to one of Claims 19 to 21 for the adjustment of the working time of a dental material.

**Revendications**

**1.** Matériaux dentaires, contenant des monomères et/ou des prépolymères contenant des groupes époxyde, qui durcissent selon un mécanisme cationique en chaîne
au moins un premier et un second systèmes d'amorçage,
ainsi qu'éventuellement des charges, des colorants, des modificateurs d'écoulement, des stabilisants, des substances délivrant des ions, des composés augmentant l'opacité aux rayons X ou d'autres agents modificateurs, dans lesquels
le premier système d'amorçage contient des boranes et/ou des hydrazones et est constitué de manière à ce que lors de la mise en contact avec de l'oxygène, il libère des espèces déclenchant la réaction de formation de polymère, le deuxième système d'amorçage contient des composés d'iodonium dissociables par voie radicalaire, et la quantité des systèmes d'amorçage est mesurée de manière à ce que les matériaux dentaires soient suffisamment aptes à l'écoulement encore au moins 10 secondes après la mise en contact avec l'oxygène et ensuite durcissent en un solide.

**2.** Matériaux dentaires selon la revendication 1, dans lesquels les matériaux dentaires durcissent après la mise en contact avec l'air.

**3.** Matériaux dentaires selon l'une quelconque des revendications 1 ou 2, dans lesquels les matériaux dentaires contiennent des monomères portant des doubles liaisons.

**4.** Matériaux dentaires selon l'une quelconque des revendications 1 ou 2, dans lesquels les matériaux dentaires contiennent des monomères et/ou des prépolymères et durcissent au moyen d'une polymérisation cationique par ouverture de cycle.

**5.** Matériaux dentaires selon l'une quelconque des revendications 1 ou 2, dans lesquels les matériaux dentaires contiennent des monomères et/ou des prépolymères contenant aussi bien des groupes époxyde que des doubles liaisons.

**6.** Matériaux dentaires selon l'une quelconque des revendications 1 ou 2, dans lesquels les matériaux dentaires contiennent des monomères et des prépolymères contenant des groupes (méth)acryliques et durcissent par un mécanisme radicalaire en chaîne.

**7.** Matériaux dentaires selon l'une quelconque des revendications 1 ou 2, dans lesquels les matériaux dentaires contiennent des monomères contenant des groupes allyle ou des monomères contenant des groupes norbornène et des composés à multiples fonctions thiol et durcissent selon un mécanisme de liaisons thiol.

**8.** Matériaux dentaires selon l'une quelconque des revendications 1 à 7, dans lesquels les matériaux dentaires contiennent des composés de diaryliodonium.

**9.** Procédé pour le durcissement de matériaux dentaires selon les revendications 1 à 8, dans lequel les matériaux dentaires sont conservés avant leur utilisation dans des récipients fermés étanches à la lumière ainsi qu'à l'oxygène, la composition dentaire est sortie de ce récipient et est mise en contact avec l'air ambiant pendant une durée de 1 à 120 secondes.

**10.** Procédé pour le durcissement de matériaux dentaires, selon les revendications 1 à 8, dans lequel l'oxygène est produit par une réaction chimique avec une surface « active » dans les matériaux dentaires.

**11.** Procédé pour le durcissement de matériaux dentaires, selon les revendications 1 à 8 et la revendication 10, dans lequel une surface « active » est produite, avant la sortie du matériau dentaire, par dopage d'une surface avec un matériau riche en oxygène.

12. Procédé pour le durcissement de matériaux dentaires, selon les revendications 1 à 8, dans lequel les matériaux dentaires sont sous forme pâteuse et sont, après leur sortie, mis en contact avec de l'air en un temps de 1 à 60 secondes sur un substrat avec un dispositif manuel tout en formant de nouvelles surfaces.

13. Procédé pour le durcissement de matériaux dentaires, selon les revendications 1 à 8, dans lequel les matériaux dentaires s'écoulent facilement et contiennent éventuellement des solvants et sont traités avec un courant d'air après leur sortie.

14. Utilisation de matériaux dentaires selon les revendications 1 à 8 pour la préparation d'un matériau, approprié pour la fixation de couronnes, bridges et inlays, le remplissage de cavités, le traitement de cavités préparées mécaniquement, le traitement de cavités, qui sont traitées au préalable par une étape de décapage, comme couche de liaison intermédiaire entre une paroi de cavité traitée au préalable et un matériau de remplissage.

15. Matériaux dentaires, contenant des monomères et/ou des prépolymères portant des groupes époxyde, qui durcissent selon un mécanisme cationique en chaîne, au moins un premier et un deuxième systèmes d'amorçage, ainsi qu'éventuellement des charges, des colorants, des modificateurs d'écoulement, des stabilisants, des substances délivrant des ions, des composés augmentant l'opacité aux rayons X ou d'autres agents modificateurs, dans lesquels le premier système d'amorçage contient des boranes et/ou des hydrazones et est constitué de manière à libérer, lors de la mise en contact avec de l'oxygène, des espèces amorçant la réaction de formation de polymères, le second système contient des composés d'iodonium dissociables par voie radicalaire et la quantité des systèmes d'amorçage est mesurée de manière à ce que les matériaux dentaires durcissent après la mise en contact avec l'oxygène en un solide, la couche de monomère non polymérisée sur la surface (couche déposée) étant inférieure à 0,2 mg/cm$^2$.

16. Matériaux dentaires selon la revendication 15, dans lesquels la quantité du système d'amorçage est mesurée de manière à ce que les matériaux dentaires après la mise en contact avec l'oxygène soient encore au moins pendant 10 secondes suffisamment aptes à l'écoulement et durcissent ensuite en une substance solide.

17. Récipient, contenant un matériau dentaire selon l'une quelconque des revendications 1 à 8 ou 15 à 16.

18. Utilisation des matériaux dentaires selon les revendications 15 ou 16 pour la préparation de couronnes et bridges provisoires.

19. Utilisation d'un système, contenant au moins un premier et un deuxième systèmes d'amorçage, comprenant au moins une substance sensible à l'oxygène, choisie parmi les boranes et/ou hydrazones et au moins un composé qui se décompose sous l'action de radicaux en formant des acides, choisi parmi des composés d'iodonium, pour le durcissement de préparations de formation de polymère.

20. Utilisation d'un système selon la revendication 19, dans laquelle le composé qui se décompose sous l'action de radicaux en formant des acides, est choisi parmi des composés de diaryliodonium.

21. Utilisation d'un système selon l'une quelconque des revendications 19 à 20, dans laquelle la préparation de formation de polymère comporte des substances contenant des époxydes.

22. Utilisation d'un système selon l'une quelconque des revendications 19-21 pour ajuster le temps de mise en oeuvre d'un matériau dentaire.